(19) Europäisches Patentamt

European Patent Office

Office européen des brevets

(11) **EP 1 048 000 B1**

(12) **EUROPÄISCHE PATENTSCHRIFT**

(45) Veröffentlichungstag und Bekanntmachung des Hinweises auf die Patenterteilung:
**28.08.2002 Patentblatt 2002/35**

(21) Anmeldenummer: **99900047.4**

(22) Anmeldetag: **13.01.1999**

(51) Int Cl.⁷: **G06F 17/00**

(86) Internationale Anmeldenummer:
**PCT/CH99/00014**

(87) Internationale Veröffentlichungsnummer:
**WO 99/036860 (22.07.1999 Gazette 1999/29)**

(54) **ERWEITERTE KARDIOGONIOMETRIE**

EXPANDED CARDIOGONIOMETRY

CARDIOGONIOMETRIE ELARGIE

(84) Benannte Vertragsstaaten:
**AT CH DE LI**

(30) Priorität: **16.01.1998 CH 10398**

(43) Veröffentlichungstag der Anmeldung:
**02.11.2000 Patentblatt 2000/44**

(73) Patentinhaber: **Sanz, Ernst, Dr. med.
3532 Zäziwil (CH)**

(72) Erfinder: **Sanz, Ernst, Dr. med.
3532 Zäziwil (CH)**

(74) Vertreter: **Frei, Alexandra Sarah
Frei Patentanwaltsbüro
Postfach 768
8029 Zürich (CH)**

(56) Entgegenhaltungen:
EP-A- 0 691 620     US-A- 4 492 235
US-A- 4 633 881     US-A- 4 974 598

• JAGER F ET AL: "A REAL-TIME PERSONAL COMPUTER BASED SYSTEM FOR ANALYSIS OF ELECTROCARDIOGRAMS" PROCEEDINGS OF THE COMPUTERS IN CARDIOLOGY MEETING, JERUSALEM, SEPT. 19 - 22, 1989, Nr. MEETING 16, 1990, Seiten 497-500, XP002050829 INSTITUTE OF ELECTRICAL AND ELECTRONICS ENGINEERS

**Beschreibung**

Die Erfindung liegt auf dem Gebiet der Vektorkardiographie und bezieht sich auf ein Verfahren in der Vektordarstellung elektrischer Grössen des Herzens, sowie auf eine elektrische Schnittstelle zur Verarbeitung bestimmter elektrischer Grössen zur Darstellung und Diagnose von Herzkrankheiten.

In der früher verwendeten Vektorkardiographie, bspw. mit Hilfe des Ableitungssystems nach Frank, wurde angenommen, dass die Ableitungssignale über ein Widerstandsnetzwerk sozusagen umgerechnet werden könnten, um daraus orthogonale Signale zu generieren. Diese Annahme war falsch. Dies ist einer von vielen Gründen, dass die Methode der Vektorkardiographie verlassen worden ist.

Die Orthogonalität der Projektionen der Herzströme, ist eine conditio sine qua non zur genauen Erfassung der Grösse der Potentiale. Ein neues Verfahren, das die Orthogonalität per constructionem sicherstellt, ist im Europäischen Patent Nr. 0'086'429 desselben Erfinders beschrieben und das Verfahren neu als Kardiogoniometrie bezeichnet worden, insbesondere weil sich diese Art von Messung und Auswertung von der alten Vektorkardiographie essentiell unterscheidet.

Eine Weiterentwicklung dieser Lehre beinhaltet die vorliegende Patentanmeldung, es handelt sich um eine Erweiterung der Kardiogoniometrie. Diese erfolgt in drei wesentlichen Punkten:

1. Die Beiziehung des Skalarwertes der anfallenden Messwerte und deren Informationswert mit Hilfe der Verwendung von Zusatzfunktionen, daraus resultierend, die genaue Bestimmung der Start- und Endstellen eines räumlichen Signalverlaufes.

2. Daraus resultierend die auf bspw. 2 ms genaue Ortung des sogenannten Nullpunktes, das heisst, des Beginns der Depolarisation und des 'J-points', dem Grenzpunkt zwischen der Depolarisation und Repolaristion des Herzens.

3. Die Unterteilung der Repolarisation in drei Abschnitte und deren exakte Abgrenzung voneinander.

Daraus resultiert die Erfassung von vielen Messwerten, die die Diagnose aufgrund von harten Daten im Gegensatz zu den statistischen Daten der Elektrokardiographie erlauben. Diese harten Daten werden über ein weiteres Programm erfasst und zu diagnostischen Zwecken über weitere Geräte (24-Std Goniometer, Herzüberwachungsgeräte, Defibrülatoren etc.) verwendet bzw. verarbeitet. Diese Daten stehen dann bereit für die Verwendung als Signale zur Steuerung von Online-Herzgeräten, als Daten zur Aufzeichnung von Diagrammen, als Daten zur Extrapolation und als Daten zur Herstellung einer vollcomputerisierten Herzdiagnostik.

Es ist deshalb das Ziel der Erfindung, diese bekannten Daten, die es ermöglichen, bisher über kardiographische Methoden nicht sichtbare pathologische Befunde zu ermitteln, derart zu verarbeiten, dass insbesondere die bekannten Mängel der Nullpunktbestimmung und der genauen Erfassung der Daten bei kleinen Potentialen weitgehend behoben werden. Dieses Verfahren ist in den Patentansprüchen definiert und hier anschliessend im Detail diskutiert.

Die Ermittlung des Nullpunktes, das heisst, die Bestimmung des Start- und Endpunktes eines räumlichen Signalverlaufes, hier sind es Vektorschleifen, ist schwierig. Es wurde versucht, in Zusammenarbeit mit dem Erfinder, eine praktikable Lösung zu finden, siehe bspw. US-Patent Nr. 4'700'712 eines Mitarbeiters des Erfinders, welches Verfahren wohl eine Verbesserung brachte, aber bei kleinem Signal-Rauschabstand oft versagte.

Die Lokalisation des Maximalvektors der ST-T Schleife und dessen allfällige Streuung, beides sehr wichtige Parameter zur Diagnose von Ischämiezonen und deren Lokalisation, ist nicht mehr mit Sicherheit möglich, wenn der Vektor (bzw. das Signal) weniger als 0,4 mV beträgt. Bei so kleinen Werten kann die Überlagerung von extrakardialen Störströmen (Muskelzittern u.a.) sowohl den durch einen Meridianwert ermittelten Maximalvektor wie auch die Streuung der Einzelwerte und endlich auch die Form der ST-T Schleife so stark verändern, dass eine sichere Diagnose nicht mehr möglich ist.

Der Start- und der Endpunkt einer QRS-Schlaufe in einem Koordinatensystem beginnen, zumindest theoretisch, im Ursprung bzw. Nullpunkt des Koordinatensystems. Bei ungünstigen elektrischen Verhältnissen ergeben sich jedoch Fehler in Form offener Schlaufen oder durch Verlagerungen aus dem Nullpunkt. Auch wenn über eine grössere Anzahl Schlaufen gemittelt wird, verfälschen solche Fehler einen Befund und sie werden in der Regel nicht entdeckt und entsprechend berücksichtigt.

Aus der Publikation US-4492235 ist es bekannt, kardiologisch ermittelte Messgrössen durch Glätten und Differenzieren für die Gewinnung von diagnoserelevanten Parametern aufzubereiten

Das erfinderische Verfahren realisiert sich in wenigen ideenreichen Verfahrensschritten: Die abgeleiteten Spannungswerte U werden nach dem vom gleichen Erfinder in der EP-0'086'429 beschriebenen Umrechnungs- und Konstruktions-Verfahren orthogonalisiert. Aus den orthogonalisierten Rohdaten werden die Vektorbeträge der Spannung U ermittelt. Man erzeugt eine der Messung der Rohdaten entsprechende zeitliche Abfolge von Vektorbeträgen in gleichbleibenden zeitlichen Abständen, bspw. 2 Millisekunden. Störende statistische Grössen werden eliminiert, indem jede Messgrösse mit einer Gruppe von unmittelbar vorangehenden und unmittelbar folgenden Messgrössen, bspw.

Gruppen von 5 bis 10 Messgrössen, zu einem korrigierten Messwert verarbeitet wird (moving average, kontinuierliche Mittelwertbildung oder gleitender Durchschnitt). Dadurch wird der Einfluss von Artefakten massgeblich verringert und die Abfolge der Vektorbeträge korrigiert. Aus den korrigierten Vektorbeträgen der Spannung U wird der Differentialquotient dU/dt errechnet und die Nulldurchgänge ermittelt.

Aus diesen, sozusagen bereinigten, Nulldurchgängen ergibt sich bspw. die genaue Abgrenzung der Teilstrecken R+, R-, ST, T+ und T- (siehe Figur 2) und damit auch der Nullpunkt von R+, der sogenannte J-point am Ende von R- und schliesslich der exakte Endpunkt der T-Welle, was ohne das erfindungsgemässe Vorgehen nicht erzielbar war.

Diese Daten stehen dann zur weiteren Verwendung bereit, bspw. als Parameter für weitere Berechnungen oder nach entsprechender Umsetzung als elektrische Signale zur Steuerung von Online-Kardiographen oder als Daten zur Aufzeichnung von Diagrammen, oder als Daten zur Extrapolation und so weiter. Als zusätzliches Hilfsverfahren kann noch der Quotient aus Differential und Menge der Vektorbeträge gebildet und dessen Werte weiterverwendet werden.

Anhand der nachfolgend aufgeführten Figuren wird das erfindungsgemässe Verfahren nun im Detail erklärt.

Fig. 1    zeigt ein beispielsweises Ablaufschema des Verfahrens.

Fig. 2    Die Diagramme A,B und C zeigen die aus dem Verfahren gewonnenen Daten zur Nullpunktbestimmung.

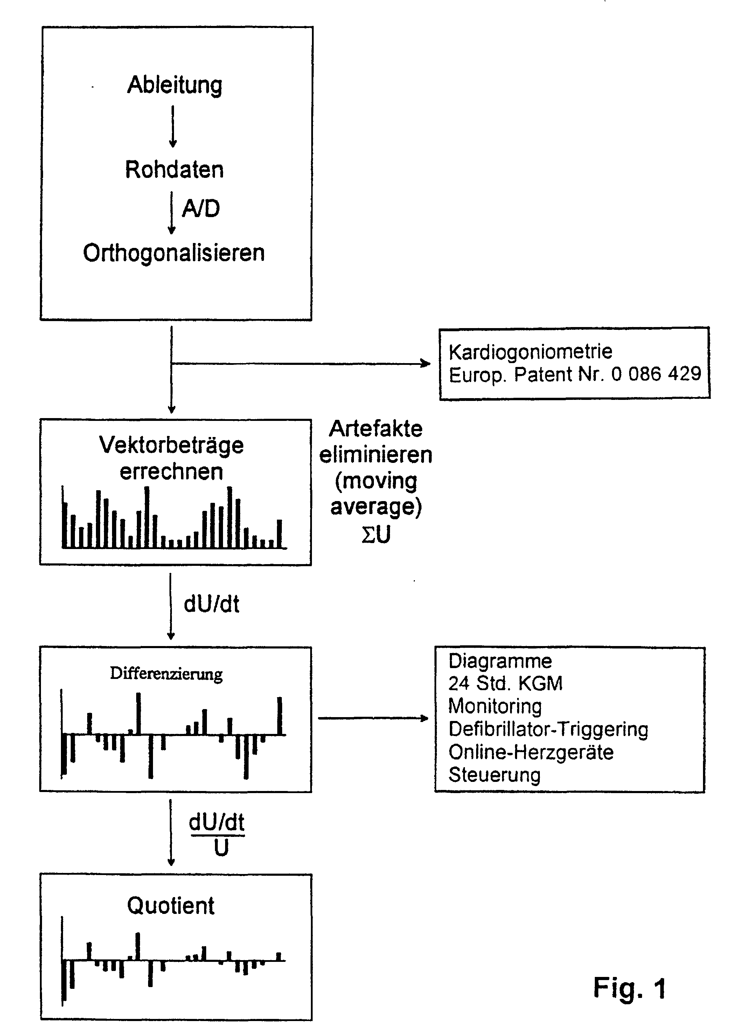

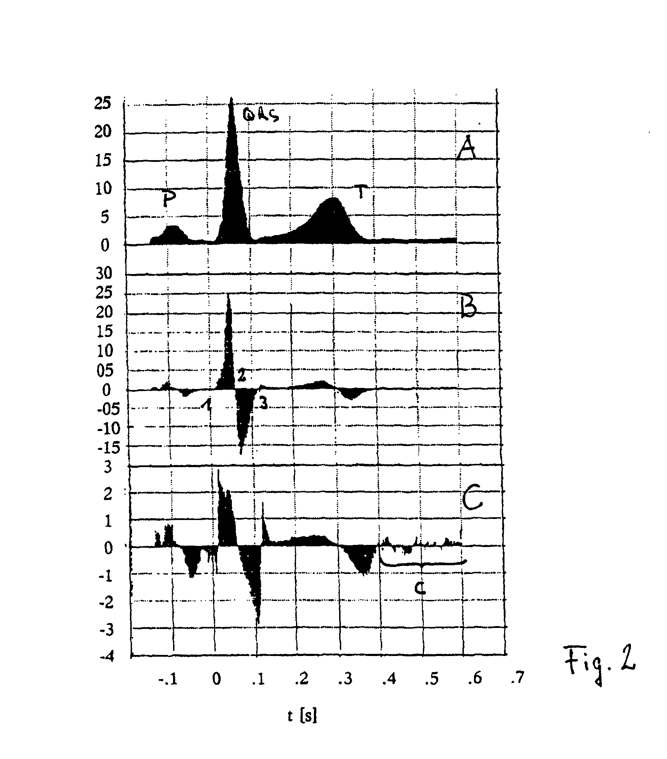

Wie Figur 1 zeigt, werden die Daten aus einer Messung in einem Zwischengerät, bspw. in einem sogenannten Satelliten, gewonnen. Die Rohdaten (Messsignale) müssen nun in mehreren Verfahrensschritten aufbereitet werden. Zuerst werden sie im Zwischengerät digitalisiert und orthogonalisiert und einem Computer zugeleitet, wo sie gemäss der oben genannten Europäischen Patentschrift Nr. 0'086'429 verarbeitet werden. Aus den gewonnenen Vektoren können verschiedene aussagekräftige Darstellungen hergestellt werden, die bekannteste davon ist die räumliche Darstellung der Vektorschleife in einem Polarkoordinatensystem. Alle diese Darstellungsformen sind darauf angewiesen, dass der sogenannte 'Nullpunkt', das ist der exakte Beginn des QRS-Signals, auf der Zeitachse bestimmt werden kann. Die nullpunktnahen Signale sind jedoch durch das Systemrauschen, hier die Unruhe der Basislinie, verdeckt und können nicht einfach sichtbar gemacht werden.

Aus diesem Grunde werden die digitalisierten und orthogonalisierten Signale in Abständen von beispielsweise einer Millisekunde registriert und die gewonnene zeitliche Funktion, in Annäherung das Integral des Potentials, wird einer glättenden, sogenannten gleitenden Durchschnittsbildung bspw. nach der nachfolgenden Formel unterzogen.

$$Y_i(\text{geglättet}) = 1/m\,(Y_{i-k} + ... + Y_i + ... + Y_{i+k}),$$

wobei i den fortlaufenden Index der einzelnen Messwerte und m = 2k+1 die Anzahl der für die Bildung des gleitenden Durchschnittes in Betracht gezogenen Messwerte darstellt, die in diesem Falle eine ungerade Zahl ist.

Für m = 2k (gerade Zahl von Messwerten) heisst die entsprechende Formel:

$$Y_i(\text{geglättet}) =$$

$$1/2\,\{1/m\,(Y_{i-k} + ... + Y_{i+k-1}) + 1/m(Y_{i-k} + ... + Y_{i+k+1})\}$$

Durch diese Glättung werden statistische Grössen und Artefakte, hier unter anderem das Basislinienrauschen, eliminiert bzw. verringert. Damit sind die elektrischen Signale praktisch eindeutig bestimmbar. Der Beginn und das Ende eines elektrischen Signals können festgelegt werden und da diese Signale zeitabhängig sind, lässt sich bspw. genau messen, wie lange das Signal dauert und nach welcher Zeitdauer das Signalmaximum erreicht ist. Ferner können die Summen der einzelnen Potentiale in den verschiedenen Zeitabschnitten einzeln ermittelt werden.

Eine Verbesserung der Nullpunktdetektion bringt das Differential der aus bspw. 1ms-Vektoren bestehenden Abfolge der zeitlich hintereinander eintreffenden elektrischen Signale und nach Umrechnung die zeitliche Abfolge deren Vektorbeträge. Durch diese Massnahme erscheinen Maxima und Minima (für den vorliegenden Fall Nullstellen) in der Abfolge der Vektorbeträge bzw. im Signalverlauf als Nulldurchgänge und können rechnerisch, elektrisch oder graphisch besonders genau und einfach festgelegt werden. Durch das Differential wird das Spektrum im Bereich der Basislinie unruhig, da sich eine ganze Anzahl irrelevanter Nullstellen bilden, besonders bei zu kleinen Potentialen, was eine Glättung des dU/dt-Diagramms vor allen Dingen nötig macht. Es hat sich ferner gezeigt, dass als Auswertungshilfe auch der Quotient aus Differential und Skalarwert der Vektorbeträge herangezogen werden kann.

Figur 2, Diagramm A zeigt die geglättete, und durch kontinuierliche Mittelwertbildung bearbeitete Abfolge der Vektorbeträge der Spannung U gleich dem V-Diagramm (V entspricht der Ableitungsspannung U, das Diagramm besteht aus der Menge bzw. Anzahl aller gemessenen Spannungen). Man erkennt drei Maxima und vier Minima. Zur Ermittlung der Zeitpunkte des QRS-Signals, welches als Vektorschlaufe im Nullpunkt beginnt und im Nullpunkt endet, wird der Differentialquotient dU/dt gebildet, welcher in Figur 2 als dU/dt-Funktion B gezeigt ist. Man erkennt hier anhand der Nulldurchgänge eindeutig, bei welchem Zeitpunkt das Signal aus der Basislinie anzusteigen beginnt (Nulldurchgang 1), wo es sein Maximum erreicht (Nulldurchgang 2) und bei welchem Zeitpunkt es endet (Nulldurchgang 3). Dasselbe erkennt man bei den kleineren Signalen auf beiden Seiten des QRS-Signals, links die P-Welle, rechts die T-Welle und in der Differential gleichartige Nulldurchgänge zeigen. Das Diagramm C in Figur 2 zeigt den Verlauf des Quotienten aus Differential und Summe der Vektorbeträge erstellt aus der geglätteten Funktion. Die Stellen der Nulldurchgänge auf der Zeitachse bleiben gleich, aber die 'peaks' der Spektren treten markanter hervor.

Dieses hier diskutierte Verfahren zur Detektion von Verläufen von kardiographischen elektrischen Signalen, und Bestimmung von weiterverwendbaren Funktionswerten auf der Zeitachse, ausgehend von den kardiogoniometrisch orthogonalisierten Messgrössen, wie im Europäischen Patent Nr. 0'086'429 beschrieben, kann in auf zwei Arten durchgeführt werden:

1) indem die in der zeitlichen Abfolge gemäss der Messung anfallenden Vektorbeträge der orthogonalisierten Messgrössen dadurch korrigiert werden, dass jede Messgrösse mit einer Gruppe unmittelbar vorangehender und unmittelbar folgender Messgrössen zu einer korrigierten Messgrösse verarbeitet wird und aus der errechneten Abfolge der korrigierten Messgrössen der Differentialquotient gebildet wird oder

2) indem aus den gemäss der Messung anfallenden zeitlichen Abfolge der Vektorbeträge von orthogonalisierten Messgrössen der Differentialquotient gebildet wird und die differenzierte Abfolge dadurch korrigiert wird, dass jede differenzierte Messgrösse mit einer Gruppe unmittelbar vorangehender und unmittelbar folgender differenzierter Messgrössen zu einer korrigierten differenzierten Messgrösse verarbeitet wird.

Die auf beiden Wegen aus der Bildung der Differentialquotienten gewonnenen und bereinigten Nullstellen werden dann zur Weiterverarbeitung bzw. Weiterbenützung bereit gestellt.

Für Untersuchungen von Teilen des Spektrums, wie beispielsweise der STT-Strecke, das heisst, die Region zwischen der rechten Flanke des QRS-Signals und dem Maximum des T-Signals, oder wie oben erwähnt, die Region rechts vom T-Signal, welche im Quotienten-Spektrum weitere Linien zeigt, können Daten aus dem Differential-Spektrum als Steuerungsparameter herangezogen werden. Die entsprechenden exakten und vor allen Dingen relevanten Nulldurchgänge des Differentialquotienten können bspw. zum exakten Triggern von Defibrillatorgeräten herangezogen werden.

## Patentansprüche

1. Verfahren zur Detektion von Verläufen von kardiographischen elektrischen Signalen und zur Bestimmung von weiterverwendbaren Funktionswerten auf der Zeitachse, ausgehend von den kardiogoniometrisch orthogonalisierten Messgrössen **dadurch gekennzeichnet, dass** die in der zeitlichen Abfolge gemäss der Messung anfallenden Vektorbeträge der orthogonalisierten Messgrössen korrigiert werden, indem jede Messgrösse mit einer Gruppe unmittelbar vorangehender und unmittelbar folgender Messgrössen zu einer korrigierten Messgrösse verarbeitet wird und aus der Abfolge der korrigierten Messgrössen der Differentialquotient gebildet wird, dass anhand der aus der Bildung der Differentialquotienten gewonnenen und bereinigten Nullstellen ein Startpunkt der Depolarisation, ein Endpunkt der Repolarisation bestimmt und der zeitliche Bereich zwischen dem Start der Depolarisation und dem Ende der Repolarisation in Abschnitte R+, R-, ST, T+ und T- aufgeteilt wird, dass die korrigierten Messgrössen über die Abschnitte R+, R-, ST, T+ und T- summiert werden und dass die Aufteilung in die genannten fünf Abschnitte und die über die Abschnitte summierten Messgrössen zur Weiterverarbeitung bereit gestellt werden.

2. Verfahren nach Anspruch 1, **dadurch gekennzeichnet, dass** aus den Messgrössen vor und nach der Differentiation Steuerungsparameter zur Steuerung von Geräten wie 24Std-Goniometer, Herzüberwachungsgeräte oder Defibrillatoren gebildet und verwendet werden.

3. Verfahren nach Anspruch 2, **dadurch gekennzeichnet, dass** aus Daten der Nulldurchgänge der differenzierten Funktion Steuerungsparameter zur Steuerung von Geräten wie 24Std-Goniometer, Herzüberwachungsgeräte oder Defibrillatoren gebildet und weiterverwendet werden.

4. Verfahren nach Anspruch 1, **dadurch gekennzeichnet, dass** aus den Funktionen der Messgrössen oder aus Teilen davon einzelne Vektorbeträge ermittelt und als Divisoren der einzelnen zugehörigen Vektorbeträge der Differentialquotienten-Funktion oder Teilen davon zur Quotientenbildung verwendet wird.

5. Verfahren nach Anspruch 4, **dadurch gekennzeichnet, dass** nach der Quotientenbildung aus Daten der Quotienten-Funktion Steuerungsparameter zur Steuerung von Geräten gebildet und verwendet werden.

**6.** Gerät zur Verarbeitung von kardiographischen elektrischen Signalen nach einem der Ansprüche 1 bis 5, **dadurch gekennzeichnet, dass** es Mittel zur Überführung der Messsignale in Messgrössen mit gleichen zeitlichen Abständen, Mittel zur Bildung eines gleitenden Durchschnittes über die Messgrössen, Mittel zur Bildung einer Abfolge von Differentialquotienten aus einer Abfolge von Messgrössen, Mittel zur Bestimmung von Nulldurchgängen in einer Abfolge von Differentialquotienten sowie Mittel zur Bildung von Summen von Messgrössen über Abschnitte zwischen zwei Nulldurchgängen in der Abfolge der entsprechenden Differentialquotienten aufweist.

**7.** Anwendung des Verfahrens nach einem der Ansprüche 1 bis 5 in einem Computer zur Erstellung von Steuersignalen und Auswertungsdaten für die Weiterverarbeitung zu einer vollcomputerisierten Herzdiagnostik.

**Claims**

**1.** A method for the detection of the courses of cardiographic electrical signals and for determining function values on the time axis for further use, the method proceeding from the cardiogoniometrically orthogonalised measuring values, **characterised in that** the vector magnitudes, of the orthogonalised measuring values occuring in temporal succession according to measurement are corrected by processing each value with a group of directly preceding and directly following values into a corrected value and the differential quotient is formed from the sequence of the corrected values, that from the zero points gained from the formation of the differential quotients, a starting point of the depolarisation and an end point of the repolarisation are determined and corrected and the temporal region between the start of the depolarisation and the end of the repolarisation is divided into sections R+, R-, ST, T+ and T-, that the corrected values are summed over the sections R+, R-, ST, T+, T- and that the division into said five sections and the values summed over the sections are made available for further processing.

**2.** A method according to claim 1, **characterised in that**, from the values before and after differentiation, control parameters for controlling apparatus such as 24-hour-goniometers, heart monitoring apparatus and defibrilators are formed and are used.

**3.** A method according to claim 2, **characterised in that**, from data of zero crossings of the differentiated function, control parameters for controlling apparatus such as 24-hour-goniometers, heart monitoring apparatus or defibrilators are formed and are used.

**4.** A method according to claim 1, **characterised in that**, from the functions of the measuring values or from parts thereof, individual vector magnitudes are determined and are used as divisors in quotient formation of the individual associated vector magnitudes of the differential quotient function or of parts thereof.

**5.** A method according to claim 4, **characterised in that** after the quotient formation, control parameters for controlling apparatus are formed from data of the quotient function and are used.

**6.** An apparatus for processing cardiographic, electrical signals according to one of claims 1 to 5, **characterised in that** it comprises means for transforming the readings into measuring values with the same temporal intervals, means for forming a flowing average over the variables, means for forming a sequence of differential quotients from a sequence of measuring values, means for determining zero crossings in a sequence of differential quotients as well as means for forming sums of measuring values over sections between two zero crossings in the sequence of the corresponding differential quotients.

**7.** The use of a method according to one of claims 1 to 5 in a computer for producing control data and evaluation data to be further processed into a fully computerised heart diagnosis.

**Revendications**

**1.** Procédé pour la détection de l'évolution de signaux électriques cardiographiques et pour la détermination sur l'axe du temps de valeurs fonctionnelles réutilisables à partir de grandeurs de mesure cardiogoniométriques orthogonalisées, **caractérisé en ce que** les valeurs vectorielles des grandeurs de mesure orthogonalisées obtenues en succession temporelle pendant la mesure sont corrigées en transformant en une valeur de mesure corrigée chaque grandeur de mesure en même temps qu'un groupe de grandeurs de mesure immédiatement précédentes et immédiatement suivantes, le quotient différentiel étant formé à partir de la succession des grandeurs de mesure corrigées, **en ce qu'**à l'aide des passages par zéro obtenus par la formation des quotients différentiels et nettoyés, on détermine un point de départ de la dépolarisation et un point final de la repolarisation, l'intervalle de temps entre le départ de la dépolarisation et la fin de la repolarisation étant divisé en tronçons R+, R-, ST, T+ et T-, **en ce que** les grandeurs de mesures corrigées sont additionnées sur les tronçons R+, R-, ST, T+ et T- et **en ce que** la division en lesdits cinq tronçons et les grandeurs de mesure additionnées sur les tronçons sont transférées aux traitements ultérieurs.

sé.

2. Procédé selon la revendication 1, **caractérisé en ce que** des paramètres de commande sont formés à partir des grandeurs de mesure avant et après la différentiation, et sont utilisés pour la commande d'appareils tels que des goniomètres sur 24 heures, des appareils de surveillance cardiaque ou des défibrillateurs.

3. Procédé selon la revendication 2, **caractérisé en ce que** des paramètres de commande sont formés à partir des données de passage par zéro de la fonction différentiée et sont réutilisés pour la commande d'appareils tels que des goniomètres sur 24 heures, des appareils de surveillance cardiaque ou des défibrillateurs.

4. Procédé selon la revendication 1, **caractérisé en ce qu'**à partir des fonctions des grandeurs de mesure ou de parties de celles-ci, on détermine des valeurs vectorielles individuelles et on les utilise comme diviseurs des valeurs vectorielles individuelles associées de la fonction de quotient différentiel ou de parties de celle-ci, pour la formation de quotients.

5. Procédé selon la revendication 4, **caractérisé en ce qu'**après la formation de quotients à partir de données de la fonction de quotient, on forme des paramètres de commande que l'on utilise pour la commande d'appareils.

6. Appareil pour le retraitement de signaux électriques cardiographiques selon l'une des revendications 1 à 5, **caractérisé en ce qu'**il présente des moyens de conversion des signaux de mesure en grandeurs de mesure à intervalles de temps égaux, des moyens pour la formation d'une moyenne mobile des grandeurs de mesure, des moyens pour la formation d'une succession de quotients différentiels à partir d'une succession de grandeurs de mesure, des moyens pour la détermination des passages par zéro dans une succession de quotients différentiels ainsi que des moyens pour la formation de sommes de grandeurs de mesure sur des tronçons situés entre deux passages par zéro dans la succession des quotients différentiels correspondants.

7. Utilisation du procédé selon l'une des revendications 1 à 5 dans un ordinateur pour l'établissement de signaux de commande et de données d'évaluation en vue de leur traitement ultérieur visant à fournir un diagnostic cardiaque entièrement informati-

Ableitung

↓

Rohdaten

↓ A/D

Orthogonalisieren

→ Kardiogoniometrie
Europ. Patent Nr. 0 086 429

Vektorbeträge
errechnen

Artefakte
eliminieren
(moving
average)
ΣU

↓ dU/dt

Differenzierung

→ Diagramme
24 Std. KGM
Monitoring
Defibrillator-Triggering
Online-Herzgeräte
Steuerung

↓ $\dfrac{dU/dt}{U}$

Quotient

Fig. 1

Fig. 2